# EUROPEAN PATENT SPECIFICATION

(11) **EP 3 407 788 B1**
(45) Date of publication and mention of the grant of the patent: **20.08.2025**
(21) Application number: 17894127.4
(22) Date of filing: 25.01.2017
(51) Int. Cl.: A61B 5/11, A61B 5/103

(54) **GAIT PROFILER SYSTEM AND METHOD**
SYSTEM UND VERFAHREN FÜR GANGPROFILIERER
SYSTÈME ET PROCÉDÉ DE PROFILAGE DE DÉMARCHE

(30) Priority: 25.01.2016 US 201662286902 P
(43) Date of publication of application: 05.12.2018
(73) Proprietor: Wistron Corporation, New Taipei City 22181 (TW)
(72) Inventor: ZOSO, Nathaniel, Québec, (QC) G2A 3E8 (CA); THIAUX, Victorien, Québec, (QC) G1X 2M8 (CA); BOUCHARD, Kevin, Québec, (QC) G1S 4L5 (CA); BILODEAU, Katia, Québec, (QC) G1S 4L5 (CA); LAVOIE, Hugues, St-Augustin-de-Desmaures (QC) G3A 3B8 (CA)
(74) Representative: Viering, Jentschura & Partner mbB Patent- und Rechtsanwälte
(86) International application number: PCT/CA2017/000016
(87) International publication number: WO 2018/137016

(56) References cited:
- WO-A1-2012/007855
- WO-A1-2015/073490
- WO-A1-2015/164706
- JP-A- 2014 208 257
- US-A1- 2011 092 860
- US-A1- 2013 310 979
- US-A1- 2015 196 403

## Description

### CROSS-REFERENCE TO RELATED APPLICATIONS

This application claims the benefits of U.S. provisional patent application No. 62/286,902 filed on January 25, 2016.

### TECHNICAL FIELD

The present disclosure relates to a gait profiler system and method for determining the gait profile of a user.

### BACKGROUND

Assistive mobility device, such as actuated orthoses, providing optimal knee assistance (i.e. energy injection during more than 99% of the user's activities) require knowledge of the state of the leg of the user, that is either a) in a stance state (i.e. in contact with the ground or b) in a swing state. WO 2015/164706 A1 describes a feedback method and wearable device to monitor and modulate knee adduction moment.

A common method of accomplishing this is using pressure sensors. However, this method has drawbacks, mainly:
- pressure sensors have to be placed into the sole of the user's shoe or create a sole that can be adapted to fit all shoes;
- the accuracy of the pressure sensors is sensitive to the user's specific stance, (depends on which part of the foot is pressed when engaged to the ground), unless a plurality of pressure sensors are used; and
- pressure sensors can be brittle and break after a short use.

Accordingly, there is a need for a gait profiler system and method for determining the gait profile of a user that overcomes the pressure sensor's drawbacks.

### SUMMARY

The present disclosure provides a gait profiler system for determining the gait profile of a user, comprising:
a first sensing system associated with a right foot of the user, including:
a first inertial sensor;
a first securing mechanism configured to secure the first sensing system to the right foot of the user;
a first set of external sensors observing a right shank, thigh and trunk spatial orientation;
a second sensing system associated with a left foot of the user, including:
   a second inertial sensor;
   a second securing mechanism configured to secure the second sensing system to the left foot of the user;
   a second set of external sensors observing a left shank, thigh and trunk spatial orientation;
   at least one processor in communication with the first and second inertial sensors and the first and second sets of external sensors, the at least one processor having an associated memory comprising instructions stored thereon, that when executed on the processor perform the steps of:
      receiving biomechanics information about the user from the first and second inertial sensors;
      receiving biomechanics information from the first and second sets of external sensors;
      generating locomotion-related information for the right foot of the user using the biomechanics information from the first inertial sensor and the first set of external sensors;
      generating locomotion-related information for the left foot of the user using the biomechanics information from the second inertial sensor and the second set of external sensors;
      calculating a locomotion state of the user (for example stance state or swing state) by merging the locomotion-related information of the right foot and left foot; and
      generating the gait profile of the user using the locomotion state of the user.

The present disclosure also provides a gait profiler system as described above, wherein the first and second sets of external sensors include a pair of inertial sensors configured to be positioned at respective right and left leg-knee or thigh-hip structures and a plurality of sensors providing information indicative of the angular positions of the right and left knee and thigh of the user.

The present disclosure further provides a gait profiler system as described above, wherein the various sensors are provided by an exoskeleton or orthotic devices worn by the user.

The present disclosure further provides a gait profiler system as described above, wherein the step of merging the locomotion-related information of the right foot and of the left foot of the user is performed using a sensor fusion algorithm comprising the sub-steps of:
determining a static state of each of the right foot and of the left foot of the user using the locomotion-related information of the right foot and of the left foot;
determining a dynamic state of each of the right foot and of the left foot of the user using the locomotion-related information of the right foot and of the left foot; and
determining the locomotion state of the user using the static state and the dynamic state of each of the right foot and of the left foot of the user.

The present disclosure still further provides a gait profiler system as described above, wherein the step of generating the gait profile of the user includes the sub-steps of:
calculating secondary gait information using at least one of the biomechanics information about the user from the first and second inertial sensors and the biomechanics information from the first and second sets of external sensors;
calculating the gait profile based on the locomotion state of the user, the locomotion state having an associated model gait profile; and
optimizing the gait profile based on the secondary gait information.

### BRIEF DESCRIPTION OF THE FIGURES

Embodiments of the disclosure will be described by way of examples only with reference to the accompanying drawings, in which:
FIG. 1 is a schematic representation of a gait profiler system;
FIG. 2 is a schematic representation of the gait profiler system in accordance with an illustrative embodiment of the present disclosure;
FIG. 3 is a flow diagram of the state calculation process in accordance with the illustrative embodiment of the present disclosure;
FIG. 4 is a flow diagram of the sensor fusion algorithm sub-steps of the state calculation process of FIG. 3; and
FIG. 5 is a flow diagram of the gait profile calculation process in accordance with the illustrative embodiment of the present disclosure.

Similar references used in different Figures denote similar components.

### DETAILED DESCRIPTION

Generally stated, the non-limitative illustrative embodiment of the present disclosure provides a system and method for determining the gait profile of a user. The gait profiler system uses sensing systems that include inertial sensors configured to be positioned at the right and left foot-ankle structure, as well as spatial orientation of lower extremity body segments (shanks, thighs, and trunk) of the person for which the gait profile is to be determined. In an illustrative embodiment, the gait profiler system uses two additional inertial sensors at the left and right leg-knee or thigh-hip structure as well as sensors providing information indicative of the angular positions of the left and right knee and thigh, which may be provided by an exoskeleton or orthotic devices worn by the user, such as described, for example, in US Patent No. 9,370,439 entitled "LOAD DISTRIBUTION DEVICE FOR HUMAN JOINTS". This determination of the gait profile of the user is performed using biomechanics information about the user from the inertial sensors combined with the knee and hip angles.

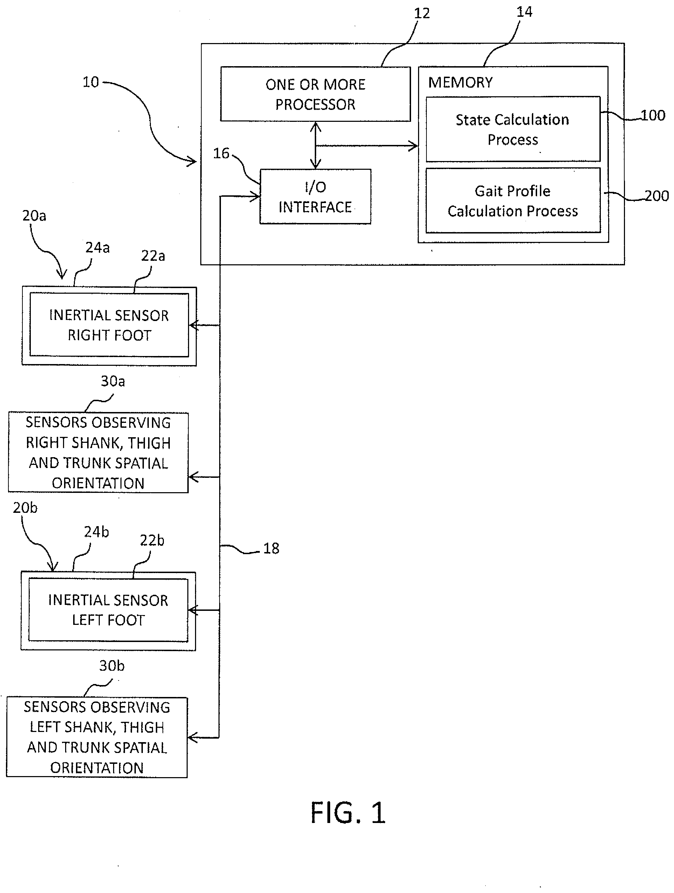

Referring to FIG. 1, the gait profiler system 10 includes one or more processor 12 with an associated memory 14 comprising instructions stored thereon, that when executed on the processor 12, perform the steps of the state calculation process 100 and the gait profile calculation process 200, which processes will be further described below, and an input/output (I/O) interface 16 for communication with a right foot 20a and a left foot 20b sensing systems and external sensors observing the right 30a and left 30b shank, thigh and trunk spatial orientation through communication link 18, which may be wired, wireless or a combination of both.

Each of the sensing systems 20a, 20b includes, respectively, an associated inertial sensor 22a, 22b (providing biomechanics information about a respective foot of the user) and a securing mechanism 24a, 24b configured to secure the sensing systems 20a, 20b, for example, right below the medial malleolus of an associated foot of the user.

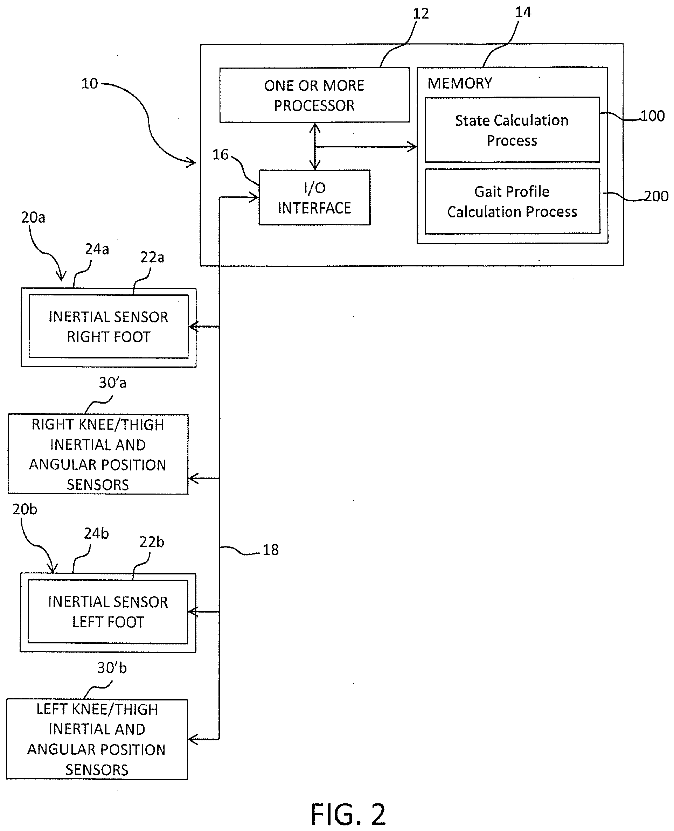

In an illustrative embodiment of the gait profiler system 10, shown in FIG. 2, the external sensors 30a, 30b take the form of right 30'a and left 30'b knee or thigh inertial and knee and hip angular positions sensors.

It is to be understood that the knee and hip angular position sensors 30'a, 30'b may take the form of any sensors providing information indicative of angular position or from which angular position may be generated as the knee and hip angles may be determine by direct measurement or deduced from biomechanics information provided by a variety of types of sensors.

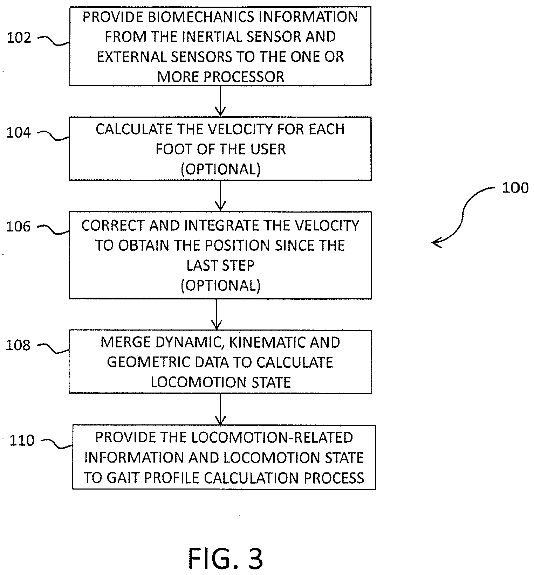

Referring to FIG. 3, there is shown a flow diagram of the state calculation process 100 executed by the one or more processor 12 (see FIGS. 1 and 2) in accordance with the illustrative embodiment of the present disclosure. Steps of the process 100 are indicated by blocks 102 to 110.

The process 100 starts at block 102 where the biomechanics information and knee and hip angles from the associated inertial sensors 20a, 20b and the external sensors 30a, 30b are provided to the one or more processor 12.

At block 104, optionally, the velocity is calculated by integrating the acceleration expressed in the global coordinates system.

At block 106, optionally, the velocity is corrected and integrated to obtain the position since the last step.

Then, at block 108, the position and velocity (if optional steps 104 and 106 are performed), acceleration, rotation and orientation are merged with a sensor fusion algorithm in order to calculate the locomotion state (i.e. stance or swing state) of the user.

Finally, at block 110, the process 100 provides the stance or swing state of the user and the the locomotion-related information of each foot of the user to the gait profile calculation process 200.

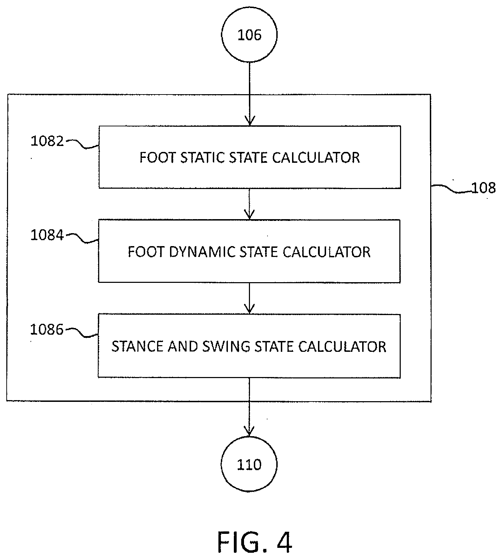

Referring to FIG. 4, there is shown a flow diagram of the sensor fusion algorithm sub-steps used in step 108 of the state calculation process 100 of FIG. 3. The sensor fusion algorithm sub-steps are indicated by blocks 1082 to 1086.

At block 1082, the static state of each of the right foot and left foot of the user is determined using the biomechanics information, i.e. is the foot in contact with the ground and is motionless or not, etc.

Then, at block 1084, the dynamic state of each of the right and left foot of the user is determined using the biomechanics information, i.e. is the foot in motion, is it part of a locomotion cycle or not, etc.

Finally, at block 1086, the algorithm determines the locomotion state (i.e. stance or swing state) of the user. To this end, the static and dynamic states of the right foot and the left foot are used (i.e. static right foot, static left foot, dynamic right foot, dynamic left foot), the various combinations of the right foot and left foot states determining if the user is in a stance or swing state. It is to be understood that other biomechanics information may be used to complement the static and dynamic states of the right foot and the left foot.

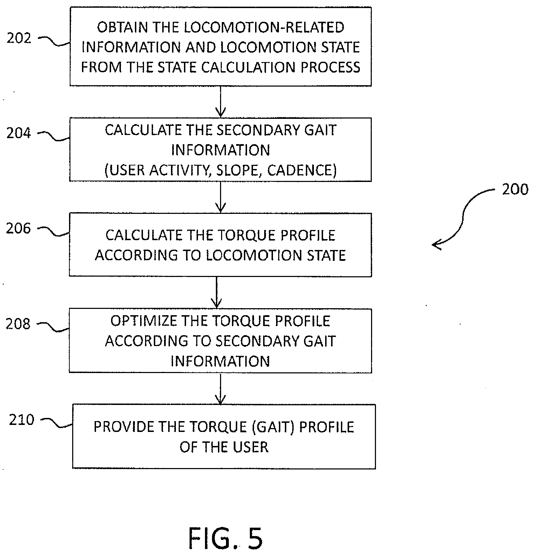

Referring now to FIG. 5, there is shown a flow diagram of the gait profile calculation process 200 executed by the one or more processor 12 (see FIGS. 1 and 2) in accordance with the illustrative embodiment of the present disclosure. Steps of the process 200 are indicated by blocks 202 to 210.

The process 200 starts at block 202 where the locomotion state (i.e. stance or swing state) of the user and the the locomotion-related information of each foot of the user is obtained from the state calculation process 100 (see FIG. 3);

At block 204, the secondary gait information such as user activity, slope, cadence, etc., is calculated from the biomechanics information and knee and hip angles.

At block 206, a torque profile is calculated based on the stance or swing state of the user. Each state is provided with a model torque profile, i.e. stance state torque and swing state torque profiles

Then, at block 208, the torque profile is optimized based on the user secondary gait information. This means that when a change of locomotion state and/or secondary gait information is detected, the torque profile is adjusted in order to limit the effects of those changes on the gait of the user.

Finally, at block 210, the process 200 provides the torque profile (i.e. gait profile) of the user.

It is to be understood that in alternative embodiments the state calculation process 100 and the gait profile calculation process 200 may be executed on a single or separate processors 12 and that the state calculation process 100 may be executed on separate processors 12 for the right and left foot of the user, the inertial sensors 20a, 20b and external sensors 30a, 30b providing their information directly to their associated processor 12.

## Claims

1. A gait profiler system for determining the gait profile of a user, comprising:
a first sensing system associated with a right foot of the user, including:
a first inertial sensor;
a first securing mechanism configured to secure the first sensing system right below the medial malleolus of the right foot of the user;
a first set of external sensors observing a right shank, thigh and trunk spatial orientation;
a second sensing system associated with a left foot of the user, including:
a second inertial sensor;
a second securing mechanism configured to secure the second sensing system right below the medial malleolus of the left foot of the user;
a second set of external sensors observing a left shank, thigh and trunk spatial orientation;
at least one processor in communication with the first and second inertial sensors and the first and second sets of external sensors, the at least one processor having an associated memory comprising instructions stored thereon, that when executed on the processor perform the steps of:
receiving biomechanics information about the user from the first and second inertial sensors;
receiving biomechanics information from the first and second sets of external sensors;
generating locomotion-related information for the right foot of the user using the biomechanics information from the first inertial sensor and the first set of external sensors;
generating locomotion-related information for the left foot of the user using the biomechanics information from the second inertial sensor and the second set of external sensors;
calculating a locomotion state of the user by merging the locomotion-related information of the right foot and of the left foot;
calculating secondary gait information using at least one of the biomechanics information about the user from the first and second inertial sensors and the biomechanics information from the first and second sets of external sensors;
calculating a gait profile based on the locomotion state of the user, the locomotion state having an associated model gait profile; and
optimizing the gait profile based on the secondary gait information.

2. The gait profiler system of claim 1, wherein the first and second sets of external sensors include a pair of inertial sensors configured to be positioned at respective right and left leg-knee or thigh-hip structures and a plurality of sensors providing information indicative of the angular positions of the right and left knee and thigh of the user.

3. The gait profiler system of claims 2, wherein the inertial sensors at the left and right leg-knee or thigh-hip structures and the plurality of sensors providing information indicative of the angular positions of the right and left knee are provided by an exoskeleton or orthotic devices worn by the user.

4. The gait profiler system of any of claims 1 to 3, wherein the biomechanics information provided by the inertial sensors include acceleration and the steps of generating locomotion-related information for the right foot and the left foot of the user include calculating a velocity by integrating the acceleration expressed in a global coordinates system.

5. The gait profiler system of claim 4, wherein the steps of generating locomotion-related information for the right foot and the left foot of the user include calculating a position by integrating the velocity.

6. The gait profiler system of claim 5, wherein the step of merging the locomotion-related information of the right foot and left foot of the user includes merging the velocity and the position.

7. The gait profiler system of any of claims 1 to 6, wherein the step of generating the gait profile of the user further uses the locomotion-related information of the right foot and of the left foot.

8. The gait profiler system of any of claims 1 to 7, wherein the step of merging the locomotion-related information of the right foot and of the left foot of the user is performed using a sensor fusion algorithm.

9. The gait profiler system of claim 8, wherein the sensor fusion algorithm comprises the sub-steps of:
determining a static state of each of the right foot and of the left foot of the user using the locomotion-related information of the right foot and of the left foot;
determining a dynamic state of each of the right foot and of the left foot of the user using the locomotion-related information of the right foot and of the left foot; and
determining the locomotion state of the user using the static state and the dynamic state of each of the right foot and the left foot of the user.

10. The gait profiler system of claim 9, wherein the sub-step of determining the locomotion state of the user further uses the locomotion-related information for the right foot and the left foot of the user.

11. The gait profiler system of claim 1, wherein the secondary gait information includes at least one of a user activity, a slope and a cadence.

12. The gait profiler system of any of claims 1 to 11, wherein the locomotion state of the user is one of a stance state and a swing state.

13. The gait profiler system of claim 1, wherein the processor further performs the steps of:
adjusting the gait profile upon detection of a change in the locomotion state or secondary gait information to limit effects of the locomotion state or secondary gait information changes on the gait of the user; and
providing the gait profile to the user.

## Patentansprüche

1. Ein Gangprofiler-System zur Bestimmung des Gangprofils eines Benutzers, umfassend:
ein erstes Sensorsystem, das einem rechten Fuß des Benutzers zugeordnet ist, umfassend:
einen ersten Trägheitssensor;
einen ersten Befestigungsmechanismus, der so eingerichtet ist, dass er das erste Sensorsystem direkt unterhalb des Innenknöchels des rechten Fußes des Benutzers befestigt;
einen ersten Satz externer Sensoren, die eine räumliche Ausrichtung des rechten Schenkels, Oberschenkels und Rumpfes beobachten;
ein zweites Sensorsystem, das einem linken Fuß des Benutzers zugeordnet ist, umfassend:
einen zweiten Trägheitssensor;
einen zweiten Befestigungsmechanismus, der so eingerichtet ist, dass er das zweite Sensorsystem direkt unterhalb des Innenknöchels des linken Fußes des Benutzers befestigt;
einen zweiten Satz externer Sensoren, die eine räumliche Ausrichtung des rechten Schenkels, Oberschenkels und Rumpfes beobachten;
mindestens einen Prozessor, der mit dem ersten und dem zweiten Trägheitssensor und dem ersten und dem zweiten Satz externer Sensoren in Verbindung steht, wobei der mindestens einen Prozessor einen zugehörigen Speicher aufweist, der darauf gespeicherte Anweisungen umfasst, welche, wenn sie auf dem Prozessor ausgeführt werden, die folgenden Schritte ausführen:
Empfangen von biomechanischen Informationen über den Benutzer von den ersten und zweiten Trägheitssensoren;
Empfangen von biomechanischen Informationen von den ersten und zweiten Sätzen externer Sensoren;
Erzeugen von Bewegungsinformationen für den rechten Fuß des Benutzers unter Verwendung der biomechanischen Informationen von dem ersten Trägheitssensor und dem ersten Satz externer Sensoren;
Erzeugen von Bewegungsinformationen für den linken Fuß des Benutzers unter Verwendung der biomechanischen Informationen von dem zweiten Trägheitssensor und dem zweiten Satz externer Sensoren;
Berechnen eines Bewegungszustands des Benutzers durch Zusammenführen der Bewegungsinformationen des rechten Fußes und des linken Fußes;
Berechnen von sekundären Ganginformationen unter Verwendung mindestens einer der biomechanischen Informationen über den Benutzer aus den ersten und zweiten Trägheitssensoren und der biomechanischen Informationen aus dem ersten und zweiten Satz externer Sensoren;
Berechnen eines Gangprofils basierend auf dem Bewegungszustand des Benutzers, wobei der Bewegungszustand ein zugehöriges Modellgangprofil aufweist; und
Optimieren des Gangprofils auf der Grundlage der sekundären Ganginformationen.

2. Das Gangprofiler-System nach Anspruch 1, wobei der erste und der zweite Satz externer Sensoren ein Paar Trägheitssensoren umfassen, die so konfiguriert sind, dass sie an den jeweiligen rechten und linken Knie- oder Oberschenkel-Hüftstrukturen der Beine angebracht sind, sowie mehrere Sensoren, die Informationen bereitstellen, welche die Winkelpositionen des rechten und linken Knies und Oberschenkels des Benutzers anzeigen.

3. Das Gangprofiler-System nach Anspruch 2, wobei die Trägheitssensoren an den linken und rechten Knie- oder Oberschenkel-Hüftstrukturen der Beine und die mehreren Sensoren, die Informationen bereitstellen, welche die Winkelpositionen des rechten und linken Knies anzeigen, von einem Exoskelett oder orthopädischen Vorrichtungen bereitgestellt werden, die vom Benutzer getragen werden.

4. Das Gangprofiler-System nach einem der Ansprüche 1 bis 3, wobei die von den Trägheitssensoren bereitgestellten biomechanischen Informationen die Beschleunigung umfassen und die Schritte zur Erzeugung von Bewegungsinformationen für den rechten Fuß und den linken Fuß des Benutzers die Berechnung einer Geschwindigkeit durch Integration der in einem globalen Koordinatensystem ausgedrückten Beschleunigung umfassen.

5. Das Gangprofiler-System nach Anspruch 4, wobei die Schritte zum Erzeugen von bewegungsbezogenen Informationen für den rechten Fuß und den linken Fuß des Benutzers das Berechnen einer Position durch Integration der Geschwindigkeit umfassen.

6. Das Gangprofiler-System nach Anspruch 5, wobei der Schritt des Zusammenführens der bewegungsbezogenen Informationen des rechten Fußes und des linken Fußes des Benutzers das Zusammenführen der Geschwindigkeit und der Position umfasst.

7. Das Gangprofiler-System nach einem der Ansprüche 1 bis 6, wobei der Schritt zum Erzeugen des Gangprofils des Benutzers ferner die bewegungsbezogenen Informationen des rechten Fußes und des linken Fußes verwendet.

8. Das Gangprofiler-System nach einem der Ansprüche 1 bis 7, wobei der Schritt des Zusammenführens der bewegungsbezogenen Informationen des rechten Fußes und des linken Fußes des Benutzers mithilfe eines Sensorfusionsalgorithmus durchgeführt wird.

9. Das Gangprofiler-System nach Anspruch 8, wobei der Sensorfusionsalgorithmus die folgenden Unterschritte umfasst:
Bestimmen eines statischen Zustands sowohl des rechten Fußes als auch des linken Fußes des Benutzers unter Verwendung der bewegungsbezogenen Informationen des rechten Fußes und des linken Fußes;
Bestimmen eines dynamischen Zustands sowohl des rechten Fußes als auch des linken Fußes des Benutzers unter Verwendung der bewegungsbezogenen Informationen des rechten Fußes und des linken Fußes; und
Bestimmen des Bewegungszustands des Benutzers unter Verwendung des statischen Zustands und des dynamischen Zustands sowohl des rechten Fußes als auch des linken Fußes des Benutzers.

10. Das Gangprofiler-System nach Anspruch 9, wobei der Unterschritt des Bestimmens des Bewegungszustands des Benutzers ferner die bewegungsbezogenen Informationen für den rechten Fuß und den linken Fuß des Benutzers verwendet.

11. Das Gangprofiler-System nach Anspruch 1, wobei die sekundäre Ganginformation mindestens eine der folgenden Informationen enthält: eine Benutzeraktivität, eine Steigung und eine Trittfrequenz.

12. Das Gangprofiler-System nach einem der Ansprüche 1 bis 11, wobei der Bewegungszustand des Benutzers entweder ein Standzustand oder ein Schwungzustand ist.

13. Das Gangprofiler-System nach Anspruch 1, wobei der Prozessor ferner die folgenden Schritte ausführt:
Anpassen des Gangprofils bei Erkennen einer Änderung des Bewegungszustandes oder sekundärer Ganginformationen, um die Auswirkungen von Änderungen des Bewegungszustandes oder sekundärer Ganginformationen auf den Gang des Benutzers zu begrenzen; und
Bereitstellen des Gangprofils für den Benutzer.

## Revendications

1. Système de profilage de démarche pour déterminer le profil de démarche d'un utilisateur, comprenant :
un premier système de détection associé à un pied droit de l'utilisateur, comprenant :
un premier capteur inertiel ;
un premier mécanisme de fixation configuré pour fixer le premier système de détection juste en dessous de la malléole médiale du pied droit de l'utilisateur ;
un premier ensemble de capteurs externes observant l'orientation spatiale du tibia, de la cuisse et du tronc droits ;
un deuxième système de détection associé au pied gauche de l'utilisateur, comprenant :
un deuxième capteur inertiel ;
un deuxième mécanisme de fixation configuré pour fixer le deuxième système de détection juste en dessous de la malléole interne du pied gauche de l'utilisateur ;
un deuxième ensemble de capteurs externes observant l'orientation spatiale du tibia, de la cuisse et du tronc gauches ;
au moins un processeur en communication avec les premier et deuxième capteurs inertiels et les premier et deuxième ensembles de capteurs externes, l'au moins un processeur ayant une mémoire associée comprenant des instructions stockées, qui, lorsqu'elles sont exécutées sur le processeur, réalisent les étapes suivantes :
réception d'informations biomécaniques sur l'utilisateur à partir des premier et deuxième capteurs inertiels ;
réception d'informations biomécaniques provenant des premier et deuxième ensembles de capteurs externes ;
génération d'informations relatives à la locomotion du pied droit de l'utilisateur à l'aide des informations biomécaniques fournies par le premier capteur inertiel et le premier ensemble de capteurs externes ;
génération d'informations relatives à la locomotion du pied gauche de l'utilisateur en utilisant les informations biomécaniques du deuxième capteur inertiel et du deuxième ensemble de capteurs externes ;
calcul d'un état de locomotion de l'utilisateur en fusionnant les informations relatives à la locomotion du pied droit et du pied gauche ;
calcul d'informations secondaires sur la démarche en utilisant au moins l'une des informations biomécaniques sur l'utilisateur provenant des premier et deuxième capteurs inertiels et des informations biomécaniques provenant des premier et deuxième ensembles de capteurs externes ;
calcul d'un profil de démarche basé sur l'état de locomotion de l'utilisateur, l'état de locomotion étant associé à un modèle de profil de démarche ; et
optimisation du profil de démarche sur la base des informations secondaires sur la démarche.

2. Système de profilage de démarche selon la revendication 1, dans lequel les premier et deuxième ensembles de capteurs externes comprennent une paire de capteurs inertiels configurés pour être positionnés sur les structures jambe-genou ou cuisse-hanche respectives droite et gauche et une pluralité de capteurs fournissant des informations indiquant les positions angulaires du genou et de la cuisse droite et gauche de l'utilisateur.

3. Système de profilage de démarche selon la revendication 2, dans lequel les capteurs inertiels au niveau des structures jambe-genou ou cuisse-hanche gauche et droite et la pluralité de capteurs fournissant des informations indiquant les positions angulaires du genou droit et gauche sont fournis par un exosquelette ou des dispositifs orthétiques portés par l'utilisateur.

4. Système de profilage de démarche selon l'une quelconque des revendications 1 à 3, dans lequel les informations biomécaniques fournies par les capteurs inertiels comprennent l'accélération et les étapes de génération des informations relatives à la locomotion pour le pied droit et le pied gauche de l'utilisateur comprennent le calcul d'une vitesse par l'intégration de l'accélération exprimée dans un système de coordonnées global.

5. Système de profilage de démarche selon la revendication 4, dans lequel les étapes de génération d'informations liées à la locomotion pour le pied droit et le pied gauche de l'utilisateur comprennent le calcul d'une position par l'intégration de la vitesse.

6. Système de profilage de démarche selon la revendication 5, dans lequel l'étape de fusion des informations relatives à la locomotion du pied droit et du pied gauche de l'utilisateur comprend la fusion de la vitesse et de la position.

7. Système de profilage de démarche selon l'une quelconque des revendications 1 à 6, dans lequel l'étape de génération du profil de démarche de l'utilisateur utilise en outre les informations relatives à la locomotion du pied droit et du pied gauche.

8. Système de profilage de démarche selon l'une quelconque des revendications 1 à 7, dans lequel l'étape de fusion des informations relatives à la locomotion du pied droit et du pied gauche de l'utilisateur est réalisée à l'aide d'un algorithme de fusion de capteurs.

9. Système de profilage de démarche selon la revendication 8, dans lequel l'algorithme de fusion des capteurs comprend les sous-étapes suivantes :
détermination d'un état statique du pied droit et du pied gauche de l'utilisateur à l'aide des informations relatives à la locomotion du pied droit et du pied gauche ;
détermination d'un état dynamique du pied droit et du pied gauche de l'utilisateur à l'aide des informations relatives à la locomotion du pied droit et du pied gauche ; et
détermination de l'état de locomotion de l'utilisateur à l'aide de l'état statique et de l'état dynamique du pied droit et du pied gauche de l'utilisateur.

10. Système de profilage de démarche selon la revendication 9, dans lequel la sous-étape de détermination de l'état de locomotion de l'utilisateur utilise en outre les informations relatives à la locomotion pour le pied droit et le pied gauche de l'utilisateur.

11. Système de profilage de démarche selon la revendication 4, dans lequel les informations secondaires sur la démarche comprennent au moins une activité de l'utilisateur, une pente et une cadence.

12. Système de profilage de démarche selon l'une quelconque des revendications 1 à 11, dans lequel l'état de locomotion de l'utilisateur est l'un parmi un état d'équilibre et un état de balancement.

13. Système de profilage de démarche selon la revendication 1, dans lequel le processeur exécute en outre les étapes suivantes :
ajuster le profil de démarche lors de la détection d'un changement dans l'état de locomotion ou dans les informations secondaires sur la démarche afin de limiter les effets des changements dans l'état de locomotion ou dans les informations secondaires sur la démarche de l'utilisateur ; et
fournir le profil de démarche à l'utilisateur.
